(19) Europäisches Patentamt
European Patent Office
Office européen des brevets

(11) Publication number: 0 223 410
A2

(12) # EUROPEAN PATENT APPLICATION

(21) Application number: 86307981.0

(22) Date of filing: 15.10.86

(51) Int. Cl.[4]: C07C 93/08 , C07C 93/14 , C07C 149/36 , C07C 143/75 , C07D 215/22 , C07D 295/08 , A61K 31/135 , A61K 31/16 , A61K 31/40 , A61K 31/47

(30) Priority: 16.10.85 GB 8525488
16.10.85 GB 8525489
16.10.85 GB 8525490
16.10.85 GB 8525491
16.10.85 GB 8525492

(43) Date of publication of application: 27.05.87 Bulletin 87/22

(84) Designated Contracting States: AT BE CH DE ES FR GB GR IT LI LU NL SE

(71) Applicant: GLAXO GROUP LIMITED
Clarges House 6-12 Clarges Street
London W1Y 8DH(GB)

(72) Inventor: Finch, Harry
19 Hensley Close
Hitchin Hertfordshire(GB)
Inventor: Lunts, Lawrence Henry Charles
10 Wormley West End
Broxbourne Hertfordshire(GB)
Inventor: Naylor, Alan
35 Layston Park
Royston Hertfordshire(GB)
Inventor: Skidmore, Ian Frederick
2 Wendover Drive
Welwyn Hertfordshire(GB)
Inventor: Campbell, Ian Baxter
5 Peregrine House The Blanes
Ware Hertfordshire(GB)

(74) Representative: Marchant, James Ian et al
Elkington and Fife High Holborn House 52/54
High Holborn
London WC1V 6SH(GB)

(54) Ethanolamine Derivatives.

(57) The invention provides compounds of the general formula (I)

EP 0 223 410 A2

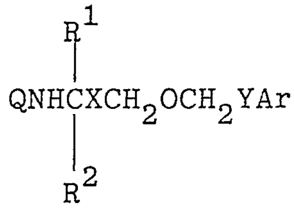

(I)

wherein

Ar represents a phenyl group optionally substituted by one or more substituents selected from halogen atoms, or the groups $C_{1-6}$alkyl, $C_{1-6}$alkoxy, nitro, $-(CH_2)_qR$ [where R is hydroxy, $-NR^3R^4$ (where $R^3$ and $R^4$ each represent a hydrogen atom or a $C_{1-4}$ alkyl group, or $-NR^3R^4$ forms a saturated heterocyclic amino group which has 5-7 ring members and optionally contains in the ring one or more atoms selected from -O-or -S-or a group -NH-or -N-($CH_3$)-), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$ alkoxy or $-NR^3R^4$), $-SR^9$ (where $R^9$ is a hydrogen atom, or a $C_{1-4}$ alkyl or phenyl group), $-SOR^9$, $-SO_2R^9$ or -CN, and q represents an integer from 0 to 3], $-(CH_2)_rR^{10}$ (where $R^{10}$ is a $C_{1-4}$ alkoxy group and r represents an integer from 1 to 3) or $-O(CH_2)_tR^{11}$ (where $R^{11}$ represents a hydroxy or $C_{1-4}$ alkoxy group and t is an integer 2 or 3), or Ar is a phenyl ring substituted by an alkylenedioxy group of formula $-O(CH_2)_pO-$where p represents an integer 1 or 2;

Q represents a group of formula

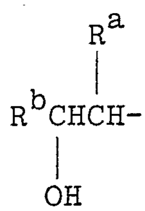 or 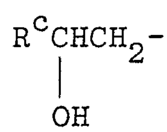

where $R^a$ represents a hydrogen atom or a $C_{1-3}$ alkyl group, $R^b$ represents

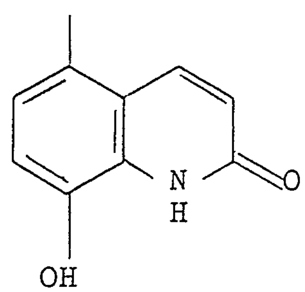

or

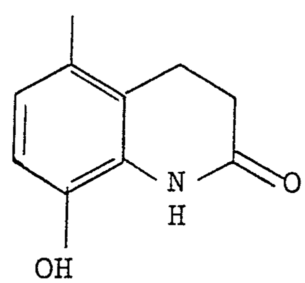

and $R^c$ represents

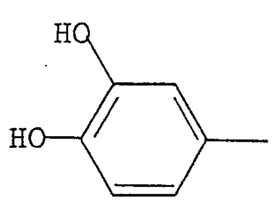 , 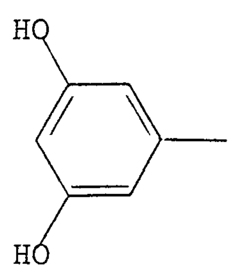 or 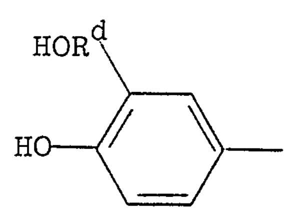

where $R^d$ represents a straight or branched $C_{2-3}$ alkylene chain; R' and $R^2$ each represent a hydrogen atom or a $C_{1-3}$ alkyl group with the proviso that the sum total of carbon atoms in R' and $R^2$ is not more than 4;

X represents a $C_{1-7}$ alkylene, $C_{2-7}$ alkenylene or $C_{2-7}$alkynylene chain; and

Y represents a bond or a $C_{1-6}$ alkylene, $C_{2-6}$ alkenylene or $C_{2-6}$ alkynylene chain with the proviso that the sum total of carbon atoms in X and Y is 2-10, and when X represents $C_{1-7}$ alkylene and Y represents a bond or $C_{1-6}$alkylene, then the group Ar does not represent an unsubstituted phenyl group or a phenyl group substituted by one or two substituents selected solely from halogen atoms or $C_{1-3}$ alkyl or $C_{1-3}$ alkoxy groups or an alkylenedioxy group $-O(CH_2)_pO-$;
and physiologically acceptable salts and solvates e.g. hydrates thereof.

The compounds have a selective stimulant action at $\beta_2$-adrenoreceptors and may be used, inter alia, in the treatment of diseases associated with reversible airways obstruction such as asthma and chronic bronchitis.

## ETHANOLAMINE DERIVATIVES

This invention relates to ethanolamine derivatives having a stimulant action at $\beta_2$-adrenoreceptors, to processes for their preparation, to pharmaceutical compositions containing them and to their use in medicine.

Ethanolamine derivatives of the general structures

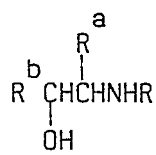

and

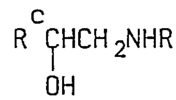

in which $R^b$ and $R^c$ represent groupings of the type described hereinafter, $R^a$ represents a hydrogen atom or an alkyl group, and R represents inter alia an alkyl, aralkyl or aryloxyalkyl group have previously been described as bronchodilators having stimulant activity at $\beta$-adrenoreceptors. We have now found a novel group of ethanolamine derivatives which differ in structure from those described previously, and have a desirable and potentially useful profile of activity.

Thus, the present invention provides compounds of the general formula (I)

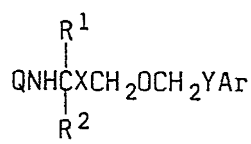

(I)

wherein

Ar represents a phenyl group optionally substituted by one or more substituents selected from halogen atoms, or the groups $C_{1-6}$alkyl, $C_{1-6}$alkoxy, nitro, $-(CH_2)_qR$ [where R is hydroxy, $-NR^3R^4$ (where $R^3$ and $R^4$ each represent a hydrogen atom or a $C_{1-4}$ alkyl group, or $-NR^3R^4$ forms a saturated heterocyclic amino group which has 5-7 ring members and optionally contains in the ring one or more atoms selected from -O- or -S-or a group -NH-or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$ alkoxy or $-NR^3R^4$), $-SR^9$ (where $R^9$ is a hydrogen atom, or a $C_{1-4}$ alkyl or phenyl group), $-SOR^9$, $SO_2R^9$, or -CN, and q represents an integer from 0 to 3], $-(CH_2)_rR^{10}$ (where $R^{10}$ is a $C_{1-4}$ alkoxy group and r represents an integer from 1 to 3) or $-O(CH_2)_tR^{11}$ (where $R^{11}$ represents a hydroxy or $C_{1-4}$ alkoxy group and t is an integer 2 or 3), or Ar is a phenyl ring substituted by an alkylenedioxy group of formula $-O(CH_2)_pO-$where p represents an integer 1 or 2;

Q represents a group of formula

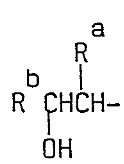

or

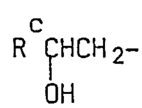

where $R^a$represents a hydrogen atom or a $C_{1-3}$ alkyl group, $R^b$ represents

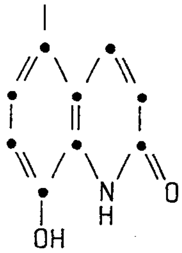

or

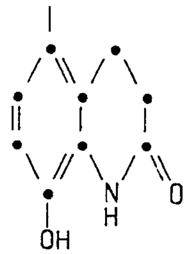

and $R^c$ represents

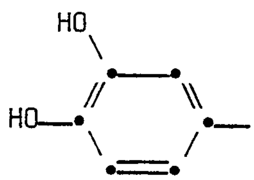

,

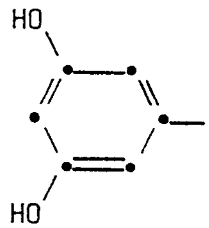

or

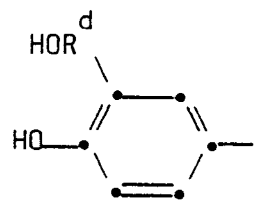

where $R^d$ represents a straight or branched $C_{2-3}$ alkylene chain;
$R^1$ and $R^2$ each represent a hydrogen atom or a $C_{1-3}$ alkyl group with the proviso that the sum total of carbon atoms in $R^1$ and $R^2$ is not more than 4;
X represents a $C_{1-7}$ alkylene, $C_{2-7}$ alkenylene or $C_{2-7}$ alkynylene chain; and
Y represents a bond or a $C_{1-6}$ alkylene, $C_{2-6}$ alkenylene or $C_{2-6}$alkynylene chain with the proviso that the sum total of carbon atoms in X and Y is 2-10, and when X represents C $_{1-7}$ alkylene and Y represents a bond or $C_{1-6}$ alkylene, then the group Ar does not represent an unsubstituted phenyl group or a phenyl group substituted by one or two substituents selected solely from halogen atoms or $C_{1-3}$alkyl or $C_{1-3}$ alkoxy groups or an alkylenedioxy group $-O(CH_2)_pO-$;
and physiologically acceptable salts and solvates (e.g. hydrates) thereof.

It will be appreciated that the compounds of general formula (I) possess one or more asymmetric carbon atoms, for example the carbon atom of the - $\underset{OH}{CH}$ -group and, when $R^1$ and $R^2$ are different groups and/or $R^a$ is an alkyl group, the carbon atoms to which these are attached, and within $R^d$ if this is a branched alkylene chain.

The compounds according to the invention thus include all enantiomers, diastereoisomers and mixtures thereof, including racemates. Compounds in which the carbon atom in the - $\underset{OH}{CH}$ -group is in the R configuration are preferred.

In the definition of general formula (I), the term alkenylene includes both cis and trans structures.

In one aspect, the invention provides compounds of formula (I) in which Q represents the group

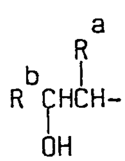

where $R^a$ represents a hydrogen atom or $C_{1-3}$ alkyl group, and $R^b$ represents

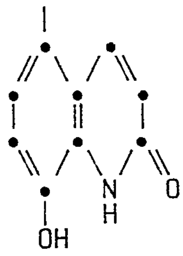

or

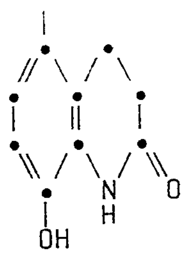
,

($R^1$, $R^2$, X, Y and Ar being as defined in formula (I)).

In another aspect, the invention provides compounds of formula (I) in which Q represents the group $R^c$ CH $CH_2$-and $R^c$ represents
OH

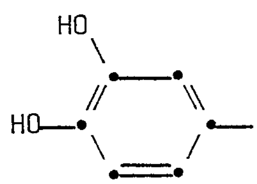
,

($R^1$, $R^2$,X,Y and Ar being as defined in formula (I).

In a further aspect, the invention provides compounds of formula (I) in which Q represents the group $R^c$ CH $CH_2$-and $R^c$ represents
OH

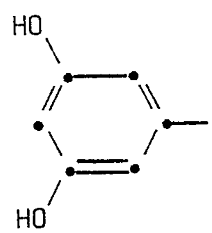
,

X represents a $C_{1-7}$ alkylene chain and Y represents a bond or a $C_{1-6}$ alkylene chain, ($R^1$, $R^2$ and Ar being as defined in formula (I)).

In yet another aspect, the invention provides compounds of formula (I) in which Q represents the group $R^c$ CH $CH_2$ and $R^c$represents
OH

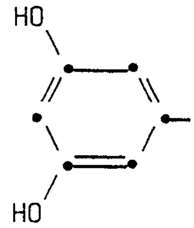 ,

and X and Y are as defined in formula (I) with the proviso that when X represents $C_{1-7}$ alkylene then Y represents $C_{2-6}$ alkenylene or $C_{2-6}$ alkynylene, ($R^1$, $R^2$ and Ar being as defined in formula (I)).

In a still further aspect, the invention provides the compounds of formula (I) in which Q represents the group $R^c\underset{\displaystyle OH}{\underset{|}{C}}H\,CH_2$-and $R^c$represents

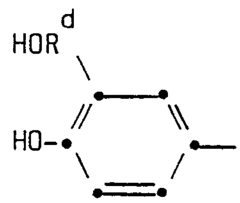 ,

where $R^d$ represents a straight or branched $C_{2-3}$ alkylene chain, ($R^1$, $R^2$, X, Y and Ar being as defined in formula (I)).

In the general formula (I), the chain X may for example contain 2 to 7 carbon atoms and may be for example $-(CH_2)_2-$, $-(CH_2)_3-$, $-(CH_2)_4-$, $-(CH_2)_5-$, $CH_2C\equiv C-$, $-(CH_2)_2CH=CH-$, $-(CH_2)_2C\equiv C-$, $-CH=CHCH_2-$, $-CH=CH(CH_2)_2$-or $-CH_2C\equiv CCH_2$. The chain Y may be for example $-CH_2-$, $-(CH_2)_2-$, $-(CH_2)_3-$, $-(CH_2)_4-$, $-(CH_2)_5-$, $-(CH_2)_6-$, $-CH=CH-$, $-C\equiv C-$, $-CH_2CH=CH-$, or $-CH_2C\equiv C-$.

In general, the total number of carbon atoms in the chains X and Y is preferably 4 to 10 inclusive and may be for example 5, 6, 7 and 8. Compounds wherein the sum total of carbon atoms in the chains X and Y is 5, 6 and 7 are particularly preferred.

One preferred group of compounds of formula (I) is that in which X is $C_{1-7}$ alkylene, Y is $C_{1-6}$ alkylene and Q, Ar, $R^1$ and $R^2$ are as defined for formula (I). Particularly interesting compounds of this type are those in which X is $-(CH_2)_3$-or $-(CH_2)_4-$, and Y is $-CH_2-$, $-(CH_2)_2$-or $-(CH_2)_3-$.

Another preferred group of compounds of formula (I) is that in which X is $-(CH_2)_3$-or $-(CH_2)_4-$, and Y is a $C_{2-3}$ alkenylene or $C_{2-3}$alkynylene group, particularly $-CH=CH-$, $-CH_2CH=CH$-or $-CH_2C\equiv C-$.

A further preferred group of compounds of formula (I) is that in which X is a $C_{3-4}$ alkenylene or $C_{3-4}$ alkynylene group, particularly $-(CH_2)_2C\equiv C$-or $-CH_2C\equiv CCH_2$-and Y is $-(CH_2)_3-$.

In the compounds of formula (I) $R^1$ and $R^2$ may each be, for example, methyl, ethyl, propyl or isopropyl groups except that if one of $R^1$ and $R^2$ is a propyl or isopropyl group, the other is a hydrogen atom or a methyl group. Thus for example $R^1$ may be a hydrogen atom or a methyl, ethyl or propyl group. $R^2$ may be for example a hydrogen atom or a methyl group. $R^1$ and $R^2$ are each preferably a hydrogen atom or a methyl group.

A preferred group of compounds is that wherein $R^1$ and $R^2$ are both hydrogen atoms, or $R^1$ is a hydrogen atom and $R^2$ is a $C_{1-3}$ alkyl group, particularly a methyl group.

$R^a$ in the compounds of formula (I) may represent for example a hydrogen atom or a methyl, ethyl or propyl group.

The group $R^d$ in general formula (I) may be for example $-(CH_2)_2-$, $-(CH_2)_3$-or $-CH(CH_3)-$, more particularly $-(CH_2)_2-$.

When $-NR^3R^4$ in compounds of formula (I) represents a saturated heterocyclic amino group, this may have 5, 6 or 7 ring members and optionally contain in the ring a heteroatom selected from -O-or -S-, or a group -NH-or $-N(CH_3)-$. Examples of such $-NR^3R^4$ groups are pyrrolidino, piperidino, hexamethylenimino, piperazino, N-methylpiperazino, morpholino, homomorpholino or thiamorpholino.

Ar may be for example a phenyl group. Examples of the substituents which may be present on the phenyl group represented by Ar include chlorine, bromine, iodine, fluorine, methyl, ethyl, methoxy, ethoxy, $-(CH_2)_qR$ [where R is hydroxy, amino, methylamino, ethylamino, dimethylamino, diethylamino, morpholino, piperidino, pyrrolidino, piperazino, N-methylpiperazino, $NHCOR^6$ (where $R^6$ is hydrogen, $C_{1-4}$ alkyl e.g.

methyl, ethyl, isopropyl or n-butyl, $C_{1-4}$alkoxy e.g. methoxy, ethoxy, isopropoxy or n-butoxy, phenyl, amino or N,N-dimethylamino), $-N(CH_3)COCH_3$, $-NR^5SO_2R^7$ (where $R^5$ represents a hydrogen atom or a methyl group, and $R^7$ represents phenyl, methyl, butyl, amino or dimethylamino), -COOH, $-COOCH_3$, $-COOCH_2CH_2CH_3$, $-CONH_2$, $-CON(CH_3)_2$, $-CON(CH_2CH_3)_2$, $-CON(CH_2CH_2CH_3)_2$,

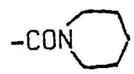 ,

$-SR^9$ (where $R^9$ is methyl, ethyl or phenyl), $-SOCH_3$, $-SO_2CH_3$, or CN and q is zero, 1, 2 or 3], $-NO_2$, $-CH_2OCH_3$, $-(CH_2)_3OCH_3$, $-O(CH_2)_2OH$, $-O(CH_2)_3OH$, $-O(CH_2)_2OCH_3$, or $-O(CH_2)_2OCH_2CH_3$.

The phenyl group represented by Ar may optionally contain one, two or three substituents, which may be present at the 2-, 3-, 4-, 5-or 6-positions on the phenyl ring.

Particular examples of a trisubstituted phenyl group represented by Ar include phenyl substituted by an amino and two methyl groups (e.g. 3,5-dimethyl-4-aminophenyl), an amino group and two chlorine atoms - (e.g. 3,5-dichloro-4-aminophenyl), or three methoxy groups (e.g. 3,4,5-trimethoxyphenyl). Particular examples of a disubstituted phenyl group represented by Ar include phenyl substituted by two hydroxyl groups - (e.g. 3,5-dihydroxyphenyl), a hydroxyl and methoxy group (e.g. 3-methoxy-4-hydroxyphenyl), or two methyl groups (e.g. 3,4-dimethylphenyl).

In one preferred group of compounds, Ar is phenyl or phenyl substituted by a fluorine atom or by a hydroxy, amino, methylamino, dimethylamino, diethylamino, piperidino, pyrrolidino, $-NHCOR^6$ [where $R^6$ is hydrogen, $C_{1-4}$ alkyl (e.g. methyl, isopropyl or n-butyl), $C_{1-4}$alkoxy (e.g. methoxy or n-butoxy), phenyl or amino], $-N(CH_3)COCH_3$, $-NHSO_2CH_3$, $-NHSO_2(CH_2)_3CH_3$, $CH_2OH$, $-CH_2NH_2$, $-COR^8$ (where $R^8$ is methoxy, propoxy or diethylamino), $-SR^9$ (where is methyl, ethyl or phenyl), $-CH_2OCH_3$, $-(CH_2)_2OH$, $-CH_2CONH_2$, or $-NO_2$ group, or by two hydroxy groups, or Ar is 3-methoxy-4-hydroxyphenyl, 3,5-dimethyl-4-aminophenyl or 3,5-dichloro-4-aminophenyl.

A further preferred group of compounds are those in which Ar represents phenyl or phenyl substituted by methoxy and/or hydroxy, or by a group selected from amino, a 5-7 membered heterocyclic amino group (e.g. pyrrolidino), $-NHSO_2R^7$ (where $R^7$ is $C_{1-4}$ alkyl e.g. methyl or butyl), $-COR^8$ (where $R^8$ is $C_{1-4}$ alkoxy e.g. methoxy, or $NR^3R^4$ where $R^3$ and $R^4$ are $C_{1-4}$ alkyl e.g. ethyl), or $-SR^9$ (where $R^9$ is $C_{1-4}$ alkyl e.g. methyl).

Preferred compounds according to the invention are:

5-[1-hydroxy-2-[[1-methyl-5-[3-[4-(1-pyrrolidinyl)phenyl]propoxy]pentyl]amino]ethyl]-1,3-benzenediol;
5-[1-hydroxy-2-[[6-[3-[4-(methylthio)phenyl]propoxy]hexyl]amino]ethyl]-1,3-benzenediol;
4-[4-[[6-[[2-(3,5-dihydroxyphenyl)-2-hydroxyethyl]amino]hexyl]oxy]butyl]-N,N-diethylbenzamide;
5-[1-hydroxy-2-[[6-[2-[4-(1-pyrrolidinyl)phenyl]ethoxy]hexyl]amino]ethyl]-1,3-benzenediol;
and their physiologically acceptable salts and hydrates.

Suitable physiologically acceptable salts of the compounds of general formula (I) include acid addition salts derived from inorganic and organic acids, such as hydrochlorides, hydrobromides, sulphates, phosphates, maleates, tartrates, citrates, benzoates, 4-methoxybenzoates, 2-or 4-hydroxybenzoates, 4-chlorobenzoates, p-toluenesulphonates, methanesulphonates, sulphamates, ascorbates, salicylates, acetates, fumarates, succinates, lactates, glutarates, gluconates, tricarballylates, hydroxynaphthalenecarboxylates e.g. 1-hydroxy-or 3-hydroxy-2-naphthalenecarboxylates, or oleates. The compounds may also form salts with suitable bases. Examples of such salts are alkali metal (e.g. sodium and potassium), and alkaline earth metal (e.g. calcium or magnesium) salts.

The compounds according to the invention have a stimulant action at $\beta_2$-adrenoreceptors, which furthermore is of a particularly advantageous profile. The stimulant action was demonstrated in the isolated trachea of the guinea-pig, where compounds were shown to cause relaxation of PGF2$\alpha$-induced contractions. Compounds according to the invention have shown an advantageous duration of action in this test.

The compounds according to the invention may be used in the treatment of diseases associated with reversible airways obstruction such as asthma and chronic bronchitis.

The compounds according to the invention may also be used for the treatment of premature labour, depression and congestive heart failure, and are also indicated as useful for the treatment of inflammatory and allergic skin diseases, glaucoma, and in the treatment of conditions in which there is an advantage in lowering gastric acidity, particularly in gastric and peptic ulceration.

The invention accordingly further provides compounds of formula (I) and their physiologically acceptable salts and solvates for use in the therapy or prophylaxis of diseases associated with reversible airways obstruction in human or animal subjects.

The compounds according to the invention may be formulated for administration in any convenient way. The invention therefore includes within its scope pharmaceutical compositions comprising at least one compound of formula (I) or a physiologically acceptable salt or solvate thereof formulated for use in human or veterinary medicine. Such compositions may be presented for use with physiologically acceptable carriers or excipients, optionally with supplementary medicinal agents.

The compounds may be formulated in conventional manner in forms suitable for administration by inhalation or insufflation, or for oral, buccal, parenteral, topical (including nasal) or rectal administration. Administration by inhalation or insufflation is preferred. The pharmaceutical compositions may be prepared by conventional means, using physiologically acceptable excipients.

A proposed daily dosage of active compound for the treatment of man is 0.005mg to 100mg, which may be conveniently administered in one or two doses. The precise dose employed will of course depend on the age and condition of the patient and on the route of administration. Thus a suitable dose for administration by inhalation is 0.005 mg to 20mg, for oral administration is 0.02mg to 100mg, and for parenteral administration is 0.01mg to 2mg for administration by bolus injection and 0.01mg to 25mg for administration by infusion.

The compounds according to the invention may be prepared by a number of processes, as described in the following wherein Q, X, Y, Ar, $R^1$ and $R^2$ are as defined for general formula (I) unless otherwise specified. It will be appreciated that certain of the reactions described below are capable of affecting other groups in the starting material which are desired in the end product; this applies especially in the reduction processes described, particularly where hydrogen and a metal catalyst are used in the preparation of compounds containing an ethylene or acetylene linkage or a hydride reducing agent is used in the preparation of compounds containing an acid, ester or amide function.

Care must therefore be taken in accordance with conventional practice, either to use reagents which will not affect such groups, or to perform the reaction as part of a sequence which avoids their use when such groups are present in the starting material. In the general processes described below for the preparation of both intermediate and end-products the final step in the reaction may be the removal of a protecting group. Suitable protecting groups and their removal are described in general process (2) below.

According to one general process (1), a compound of general formula (I) may be prepared by alkylation. Conventional alkylation procedures may be used.

Thus, for example, in one process (a), a compound of general formula (I) in which $R^1$ is a hydrogen atom may be prepared by alkylation of an amine of general formula (II)

$QNR^{12}R^{13}$ (II)

(where $R^{12}$ is a hydrogen atom or a protecting group, $R^{13}$ is a hydrogen atom, and Q is

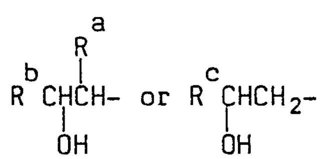

where $R^a$, $R^b$ and $R^c$ are as defined in formula (I), with any hydroxyl group(s) in $R^b$or $R^c$ optionally protected) followed by removal of any protecting group where present.

The alkylation (a) may be effected using an alkylating agent of general formula (III):

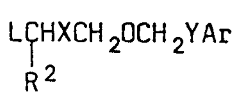

(III)

(wherein L represents a leaving group, for example a halogen atom such as chlorine, bromine or iodine, or a hydrocarbylsulphonyloxy group such as methanesulphonyloxy or p-toluenesulphonyloxy).

The alkylation is preferably effected in the presence of a suitable acid scavenger, for example inorganic bases such as sodium or potassium carbonate, organic bases such as triethylamine, diisopropylethylamine or pyridine, or alkylene oxides such as ethylene oxide or propylene oxide. The reaction is conveniently effected in a solvent such as acetonitrile or an ether e.g. tetrahydrofuran or dioxan, a ketone e.g. butanone or methyl isobutyl ketone, a substituted amide e.g. dimethylformamide or a chlorinated hydrocarbon e.g. chloroform at a temperature between ambient and the reflux temperature of the solvent.

According to another example (b) of an alkylation process, a compound of general formula (I) in which $R^1$ represents a hydrogen atom may be prepared by alkylation of an amine of general formula (II), as previously defined except that $R^{13}$ is a hydrogen atom or a group convertible thereto under the reaction conditions, with a compound of general formula (IV):

$R^2COXCH_2OCH_2YAr$ (IV)

in the presence of a reducing agent, followed when necessary by removal of any protecting groups.

Examples of suitable $R^{13}$ groups convertible into a hydrogen atom are arylmethyl groups such as benzyl, α-methylbenzyl and benzhydryl.

Suitable reducing agents include hydrogen in the presence of a catalyst such as platinum, platinum oxide, palladium, palladium oxide, Raney nickel or rhodium, on a support such as charcoal, using an alcohol, e.g. ethanol or an ester e.g. ethyl acetate or an ether e.g. tetrahydrofuran, or water, as reaction solvent, or a mixture of solvents, e.g. a mixture of two or more of those just described at normal or elevated temperature and pressure, for example from 20 to 100°C and from 1 to 10 atmospheres.

Alternatively when one or both of $R^{12}$ and $R^{13}$ are hydrogen atoms, the reducing agent maybe a hydride such as diborane or a metal hydride such as sodium borohydride, sodium cyanoborohydride or lithium aluminium hydride. Suitable solvents for the reaction with these reducing agents will depend on the particular hydride used, but will include alcohols such as methanol or ethanol, or ethers such as diethyl ether or tert-butyl methyl ether, or tetrahydrofuran.

When a compound of formula (II) where $R^{12}$ and $R^{13}$ are each hydrogen atoms is used, the intermediate imine of formula (V) may be formed:

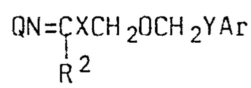

(V)

(wherein Q is as defined for formula (II)).

Reduction of the imine using the conditions described above, followed, where necessary, by removal of any protecting groups, gives a compound of general formula (I).

Where it is desired to use a protected intermediate of general formula (II) it is particularly convenient to use hydrogen and a metal catalyst as described above with protecting group $R^{12}$ and protected hydroxyl group(s) within $R^b$ and $R^c$ which are capable of being converted into hydrogen atom(s) under these reducing conditions, thus avoiding the need for a separate deprotection step. Suitable protecting groups of this type include arylmethyl groups such as benzyl, benzhydryl and α-methylbenzyl.

In another general process (2), a compound of general formula (I) may be obtained by deprotection of a protected intermediate of general formula (VI)

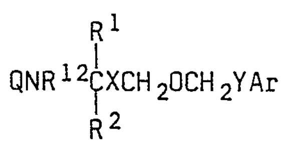

(VI)

(where $R^{12}$ and Q are as defined in formula (II) and either $R^{12}$ is a protecting group and/or at least one of the hydroxyl group(s) in $R^b$or $R^c$ is protected).

The protecting groups may be any conventional protecting groups, for example as described in "Protective Groups in Organic Chemistry", Ed. J.F.W. McOmie (Plenum Press, 1973). Examples of suitable hydroxyl protecting groups within the groups $R^b$ and $R^c$ are aralkyl groups such as benzyl, diphenylmethyl or triphenylmethyl and tetrahydropyranyl. Examples of suitable amino protecting groups represented by $R^{12}$ are aralkyl groups such as benzyl, α-methylbenzyl, diphenylmethyl or triphenylmethyl, and acyl groups such as trichloroacetyl or trifluoroacetyl.

The deprotection to yield a compound of general formula (I) may be effected using conventional techniques. Thus for example, an aralkyl group may be cleaved by hydrogenolysis in the presence of a metal catalyst (e.g. palladium on charcoal). When a hydroxyl group is protected as tetrahydropyranyl this may be cleaved by hydrolysis under acidic conditions. Acyl groups represented by $R^{12}$ may be removed by hydrolysis, for example with a base such as sodium hydroxide, or a group such as trichloroacetyl may be removed by reduction with, for example, zinc and acetic acid.

In another general process (3), a compound of general formula (I) may be prepared by reduction. Thus, for example, a compound of general formula (I) may be prepared by reducing an intermediate of general formula (VII):

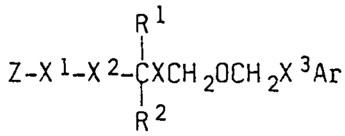

(VII)

(where Z represents $R^b$ or $R^c$ as defined in formula (I) with any hydroxyl group(s) in $R^b$ or $R^c$optionally protected, and at least one of $X^1$, $X^2$ and $X^3$ represents a reducible group and/or Ar contains a reducible group, and the other(s) take the appropriate meaning as follows, which is $X^1$ is -CH(OH)-, $X^2$ is $-CHR^a\ NR^{12}$ - (when Z is $R^b$) or $-CH_2NR^{12}$ (when Z is $R^c$) (where $R^{12}$ is as defined in formula II), $X^3$ is Y where Y is a bond or a $C_{1-6}$ alkylene chain, and Ar is as defined in formula (I), followed where necessary by removal of any protecting groups.

Suitable reducible groups include those wherein $X^1$ is a group >C=O, $X^2$ is a group $-CHR^aNY^1$-(when Z is $R^b$) or $-CH_2NY^1$-(when Z is $R^c$) (wherein $Y^1$ represents a group convertible to hydrogen by catalytic hydrogenation, for example an arylmethyl group such as benzyl, benzhydryl or α-methylbenzyl), $X^3$ represents Y where Y is a $C_{2-6}$ alkenylene or $C_{2-6}$ alkynylene chain, and Ar is a phenyl group substituted by a nitro group. In one convenient aspect of the reduction process, any hydroxyl group(s) in $R^b$ or $R^c$ may be protected as a group which is convertible to hydrogen under the reducing conditions employed and may be for example an arylmethyl group such as benzyl, benzhydryl or α-methylbenzyl.

The reduction may be effected using reducing agents conveniently employed for the reduction of ketones, protected amines, alkenes, alkynes and nitro groups.

Thus, for example, when the phenyl group Ar contains a nitro substituent, this may be reduced to an amino group using hydrogen in the presence of a catalyst as previously described for process (1) part (b).

When $X^1$ in general formula (VII) represents a >C=O group this may be reduced to a -CH(OH)-group using hydrogen in the presence of a catalyst as previously described for process (1) part (b). Alternatively, the reducing agent may be, for example, a hydride such as diborane or a metal hydride such as lithium aluminium hydride, sodium bis(2-methoxyethoxy) aluminium hydride, sodium borohydride or aluminium hydride. The reaction may be effected in a solvent, where appropriate an alcohol e.g. methanol or ethanol, or an ether such as tetrahydrofuran, or a halogenated hydrocarbon such as dichloromethane.

When $X^2$ in general formula (VII) represents a group $CHR^a\ NY^1$-or $-CH_2NY^1$-, this may be reduced to a $-CHR^aNH$-or -CHNH-group; when $X^3$ represents Y where Y is an alkenylene or alkynylene chain, this may be reduced to an alkylene chain; and/or when Ar contains a nitro substituent, this may be reduced to an amino group. These reductions may be effected using hydrogen in the presence of a catalyst as previously described for process (1) part (b).

In a further example of reduction process (3) a compound of formula (I) in which Q represents the group $R^bCH(OH)CHR^a$-and $R^b$is the group

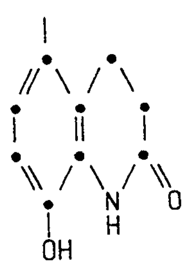

may be prepared by reducing a corresponding compound of formula (I) in which $R^b$ is the group

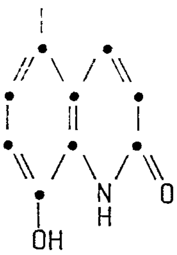

using for example hydrogen in the presence of a catalyst as previously described for process (1) part (b).

In the general processes described above, the compound of formula (I) obtained may be in the form of a salt, conveniently in the form of a physiologically acceptable. salt. Where desired, such salts may be converted to the corresponding free acids using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or iso-propanol.

Physiologically acceptable salts may also be prepared from other salts, including other physiologically acceptable salts, of the compounds of general formula (I), using conventional methods.

When a specific enantiomer of a compound of general formula (I) is required, this may be obtained by resolution of a corresponding racemate of a compound of general formula (I) using conventional methods.

Thus, in one example an appropriate optically active acid may be used to form salts with the racemate of a compound of general formula (I). The resulting mixture of isomeric salts may be separated for example by fractional crystallisation, into the diastereoisomeric salts from which the required enantiomer of a compound of general formula (I) may be isolated by conversion into the required free base.

Alternatively, enantiomers of a compound of general formula (I) may be synthesised from the appropriate optically active intermediates using any of the general processes described herein.

Specific diastereoisomers of a compound of formula (I) may be obtained by conventional methods, for example by synthesis from an appropriate asymmetric starting material using any of the processes described herein, or by conversion of a mixture of isomers of a compound of general formula (I) into appropriate diastereoisomeric derivatives e.g. salts which can then be separated by conventional means e.g. by fractional crystallisation.

Suitable methods for preparing the intermediate compounds used in the above general processes are described below. In the following discussion, Ar, $R^1$, $R^2$, $R^{12}$, $R^{13}$, Q, X, Y, X', $X^2$, $X^3$, $X^4$, Y', Z and L are as defined above except where otherwise indicated. Any hydroxyl groups in $R^b$ or $R^c$ may optionally be protected. "Hal" represents a halogen atom. Where an intermediate with protected hydroxyl and/or amino groups is desired, this may be obtained using conventional protection methods, for example those described by McOmie (see process (2) above). In addition, any substituent in Ar may be derived from a precursor substituent convertible into the required substituent by conventional methods.

Intermediate compounds of general formula (VII) for use in general process (3) may be prepared by a number of processes.

Thus for example intermediates of general formula (VII) in which X' is a group $>C=O$ may be prepared from a haloketone of formula $R^bCOCHR^aHal$ or $R^cCOCH_2Hal$ by reaction with an amine of general formula (VIII)

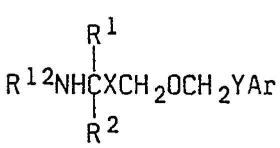

(VIII)

(where $R^{12}$ is a hydrogen atom or a group convertible thereto by catalytic hydrogenation).

The reaction may be effected in a cold or hot solvent, for example tetrahydrofuran, tert -butyl methyl ether, dioxan, chloroform, dimethylformamide, acetonitrile or a ketone such as butanone or methylisobutylketone, or an ester for example ethyl acetate, preferably in the presence of a base such as diisopropylethylamine, sodium carbonate or other acid scavenger such as propylene oxide.

Intermediates of general formula (VII) in which X' is a group $>C=O$ may be reduced to the corresponding intermediate in which X' is a group -CH(OH)-using for example a metal hydride such as sodium borohydride in a solvent e.g. ethanol.

Intermediates of formula (VII) in which $X^3$ is an alkenylene or alkynylene chain may be prepared by reacting an oxirane of formula (IX)

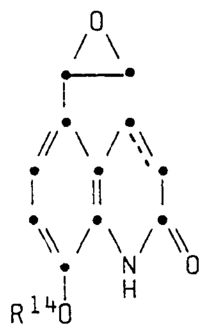

(IX)

(where $R^{14}$ represents a hydrogen atom or a protecting group) with an amine of formula (VIII) ( in which Y represents an alkenylene of alkynylene chain).

The reaction may be effected in the presence of a suitable solvent, for example an alcohol such as methanol, at a temperature from ambient to reflux.

The haloketones $R^bCOCHR^aHal$ and $R^cCOCH_2Hal$ (in which any hydroxyl substituent(s) in $R^b$ or $R^c$ may optionally be protected), the amines of formula (II), and the oxiranes of formula (IX) are either known compounds or may be prepared by methods analogous to those used for the preparation of known compounds.

Intermediates of formulae (III), (IV) and (VIII) are either known compounds or may be prepared by methods analogous to those described for the preparation of known compounds. Suitable methods are described in UK Patent Specifications Nos. 2140800A and 2159151A, and in the exemplification included hereinafter.

The following examples illustrate the invention. Temperatures are in °C. 'Dried' refers to drying using magnesium sulphate or sodium sulphate except where otherwise stated. Thin layer chromatography (t.l.c.) was carried out over $SiO_2$, and flash column chromatography (FCC) on silica (Merck 9385), using, unless otherwise stated, one of the following solvent systems: System A, cyclohexane-diethyl ether; System B, ethyl acetate-cyclohexane; System C, toluene-ethanol-0.88 ammonia solution; System D, hexane-diethyl ether, System E, hexane-ethyl acetate; System F, ethyl acetate-methanol-triethylamine; System G, ethyl acetate-methanol-0.88 ammonia solution; System H, toluene-ethanol-triethylamine. The following abbreviations are used: THF-tetrahydrofuran; DMF-dimethylformamide; BTPC -bis(triphenylphosphine)palladium(II)-chloride; TAB -tetra-n-butylammonium sulphate; DEA-diisopropylethylamine; Pd-C -palladium on charcoal; PdO-C -palladium oxide on carbon; Pt-C -platinum on charcoal; PtO -platinum oxide.

## Intermediate 1

### 1-[3-[(6-Bromohexyl)oxy]propyl]-4-(methylthio)benzene

A mixture of 4-(methylthio)benzenepropanol (5.0g), 1,6-dibromohexane (17.0g), TAB (0.4g) and aqueous sodium hydroxide (50% w/v, 20ml) was stirred for 20h, diluted with water (30ml), and extracted with ether - (2 x 100ml). The dried extract was evaporated and the residue was purified by FCC eluting with cyclohexane followed by System A (19:1) to give the title compound as a colourless oil (7.0g). T.l.c. (System A 9:1) Rf 0.5.

Intermediate 2

(E)-1-[4-[(6-Bromohexyl)oxy]-1-butenyl]-3-methoxy-4-(phenylmethoxy)benzene

as a colourless oil (11.2g), t.l.c. (System A 3:1) Rf 0.43 was prepared according to the method of Intermediate 1 from Intermediate 4 (12.0g), 1.6-dibromohexane (41.2g), 50% w/v aqueous sodium hydroxide solution (68mℓ) and TAB (1.44g), except that most of the excess dibromide was distilled off before purification by FCC eluting with System A (5:1).

Intermediate 3

N-[6-[4-(4-Nitrophenyl)butoxy]hexyl]benzenemethanamine

1-[4-[(6-Bromohexyl)oxy]butyl]-4-nitrobenzene (8.0g) was added dropwise to benzylamine (30ml) at 110°. The solution was heated at 110-120° for 2h, treated with hydrochloric acid (2M; 200ml), and extracted with ethyl acetate (2 × 150ml). The organic extract was washed with aqueous sodium carbonate (150ml) and brine (150ml), dried and evaporated. The residue was purified by FCC eluting with ether to give the title compound as a pale yellow oil (5.9g). T.l.c. Ether Rf 0.15.

Intermediate 4

(E)-4-[3-Methoxy-4-(phenylmethoxy)phenyl]-3-buten-1-ol

n-Butyllithium (1.55M in hexane, 194mℓ) was added dropwise to a stirred suspension of (3-hydroxypropyl)triphenylphosphonium bromide (60.3g) in dry THF (375mℓ) cooled to 0° under nitrogen. The resulting blood-red solution was stirred at 0° for 15 min and then a solution of 3-methoxy-4-(phenylmethoxy)benzaldehyde (36.3g) in dry THF (50mℓ) was added dropwise over 15 min. The mixture was stirred at 0° for 30 min, allowed to warm up to room temperature, stirred for a further 2h and then the reaction quenched by the addition of 2N hydrochloric acid (100mℓ). The THF was removed in vacuo at 40°, the aqueous residue extracted with ethyl acetate (350mℓ) and the organic layer washed with 2N HCl - (200mℓ). The aqueous phase was extracted with further ethyl acetate (150mℓ), the organic layers combined, washed with 8% sodium bicarbonate solution (200mℓ) and dried. Concentration afforded a product which was purified by FCC eluting with System B (1:2) yielding the title compound as a cream powder (14.5g) m.p. 57-61°

Intermediate 5

1-[3,5-Bis(phenylmethoxy)phenyl]-2-[6-[(4-nitrophenyl)butoxy]hexyl]ethanone

A solution of 2-bromo-1-[3,5-bis(phenylmethoxy)phenyl]ethanone (2.45g), Intermediate 3 (2.3g) and DEA (0.775g) in methylene chloride (20ml) was kept at room temperature for 16h, added to ether (150ml), filtered, and the filtrate evaporated. The residue was purified on a column of silica (170ml) eluting with System A (3:1) to give the title compound as a pale yellow oil (3.2g). T.l.c. (System A 1:1) Rf 0.4.

Intermediate 6

(E)-5-[1-Hydroxy-2-[[6-[[4-[[3-methoxy-4-(phenylmethoxy)phenyl]-3-butynyl]oxy]hexyl]amino]ethyl]-1,3-benzenediol

Intermediate 2 (1.79g) was added dropwise over 15 min to a stirred solution of 5-[2-amino-1-hydroxyethyl]-1,3-benzenediol (1.01g) and DEA (1.55g) in DMF (20mℓ) at 80° under nitrogen. The mixture was stirred at 80° for a further 2h, the solvent removed in vacuoat 60° and the residual oil partitioned between water (75mℓ) and ethyl acetate (100mℓ). The organic phase was dried and evaporated to yield a product which was purified by FCC eluting with System C (39:10:1) to give the title compound as a viscous colourless oil (1.13g) T.l.c. (System C 39:10:1) Rf 0.24.

Intermediate 7

6-[3-[4-(1-Pyrrolidinyl)phenyl]propoxy]-2-hexanone

(i) 3-[(4-Bromobutyl)oxy]-1-propyne

A mixture of 2-propyn-1-ol (10g), 1,4-dibromobutane (60ml), 50% aqueous sodium hydroxide (60ml) and TAB (2g) was stirred vigorously overnight. Water (250ml) was added and the mixture was extracted with ether (2 × 200ml). The organic extracts were dried and concentrated to a yellow oil which was purified by FCC (hexane → System D 19:1) to give the title compound as a colourless oil (19.7g). T.l.c. (System D 19:1) Rf 0.37.

(ii) 1-[4-[3-[(4-Bromobutyl)oxy]-1-propynyl]phenyl]pyrrolidine

A mixture of 1-(4-iodophenyl)pyrrolidine (22.8g), the product of stage (i) (16.0g), BTPC (1.5g) and copper - (I) iodide (150mg) in DEA (125mℓ) and THF (125mℓ) was stirred under nitrogen for 18h. The dark mixture was treated with ether (250mℓ), the precipitate was removed by filtration and the filtrate was concentrated to a black oil which was purified by FCC (hexane → System D 9:1) to give the title compound as a pale yellow oil (3.0g) T.l.c. (System D 9:1) Rf 0.24.

(iii) 1-[4-[3-[(4-Bromobutyl)oxy]propyl]phenyl]pyrrolidine

The product of stage (iii) (6.7g) was hydrogenated over pre-reduced 10% PdO-C in ether-THF (1:1, 60mℓ). The catalyst was removed by filtration through hyflo and the solvent was evaporated to leave the title compound as a pale brown semi-solid (6.2g) T.l.c. (System D 9:1) Rf 0.27.

(iv) 1-[4-[3-[4-(2-Methyl-1,3-dithian-2-yl)butoxy]propyl]phenyl]pyrrolidine

n-Butyllithium (1.5M in hexane, 12mℓ) was added over 5 min to a stirred solution of 2-methyl-1,3-dithiane - (2.4g) in dry THF (30mℓ) at 70° under nitrogen. The yellow solution was then stirred at -30° to -20° for 2h, cooled to -78° and treated with a solution of the product of stage (iii) (6.1g) in THF (25ml). The solution was stirred at room temperature overnight, the solvent was evaporated and the residue was purified by FCC (hexane → System D 9:1) to give the title compound as a pale yellow oil (3.2g) T.l.c. (System D 9:1) Rf 0.18.

(v) 6-[3-[4-(1-Pyrrolidinyl)phenyl]propoxy]-2-hexanone

A solution of the product of stage (iv) (3.2g) in THF (50mℓ) was added to a stirred suspension of mercury - (II) chloride (8.5g) and calcium carbonate (3.2g) in methanol-water (9:1, 50 mℓ) and the mixture was stirred at reflux for 1h. The reaction was filtered through hyflo, the filtrate was concentrated in vacuo and the resulting oil was dissolved in chloroform (50mℓ). The resulting precipitate was removed by filtration, the solvent was evaporated and the residue was purified by FCC (System D 19:1 → 4:1) to give the title compound as a crystalline mass (1.4g) m.p. 30-31°.

Intermediate 8

3,5-Bis(phenylmethoxy)-2-[[(phenylmethyl)[6-[4-(1-pyrrolidinyl)phenyl]ethoxy]hexyl]amino]methyl]-benzenemethanamine

A solution of 1-[3,5-bis(phenylmethoxy)phenyl]-2-bromoethanone (1.29g) in dry DMF (10mℓ) was added dropwise to a solution of N-[6-[2-[4-(1-pyrrolidinyl)phenyl]ethoxy]hexyl]benzenemethanamine (1.2g) and DEA (0.41g) in dry DMF (12mℓ) under nitrogen. The mixture was stirred at room temperature under nitrogen overnight, the solvent was evaporated and the residue was dissolved in absolute ethanol (30mℓ).

The solution was cooled in an ice bath and treated portionwise with sodium borohydride (1.3g) under nitrogen. After 5h the solution was brought to room temperature, stirred for a further 10min then concentrated to a yellow foam. The foam was partitioned between ethyl acetate (50mℓ) and water (50mℓ) and the organic layer washed with brine (50mℓ), dried and concentrated to a yellow oil which was purified by FCC eluting with System E to give the title compound as a yellow oil (1.35g). T.l.c. (System E 2:1) Rf 0.4.

Intermediate 9

N-[4-[3-[[6-[(Phenylmethyl)amino]hexyl]oxy]propyl]phenyl]butanesulphonamide

(i) N-[4-[3-[(6-Bromohexyl)oxy]-1-propynyl]phenyl]butanesulphonamide

A mixture of N-(4-iodophenyl)butanesulphonamide (2.01g), 1-bromo-6-[(2-propynyl)oxy]hexane (1.3g), BTPC (80mg), copper (I) iodide (10mg) and DEA (6.5mℓ) in THF (6.5mℓ) was stirred under nitrogen for 18h. The mixture was diluted with ether (50mℓ) and filtered. The filtrate was evaporated in vacuo to give a dark brown oil which was purified by FCC eluting with System E (4:1) to give the title compound as a colourless oil (1.17g). T.l.c. (System E 4:1) Rf 0.1.

(ii) N-[4-[3-[(6-Bromohexyl)oxy]propyl]phenyl]butanesulphonamide

A solution of the product of stage (i) (1.32g) in absolute ethanol (70mℓ) was hydrogenated over a pre-reduced 10% PdO-C catalyst (250mg) in absolute ethanol (10mℓ). The mixture was filtered through hyflo and evaporated in vacuo to give the title compound as a yellow oil (1.06g). T.l.c. (System C 40:10:1) Rf 0.67.

(iii) N-[4-[3-[[6-[(Phenylmethyl)amino]hexyl]oxy]propyl]phenyl]butanesulphonamide

The product of stage (ii) (0.85g) was added dropwise over 5 min to stirred benzylamine (1.17g) at 120° under nitrogen. The solution was stirred at 120° under nitrogen for 2h, cooled and diluted with dichloromethane (100mℓ). The mixture was washed with 2N hydrochloric acid (40mℓ), the aqueous phase was re-extracted with further dichloromethane (2 x 40mℓ), and the combined organic extracts were washed with 8% sodium bicarbonate solution (80mℓ), dried and evaporated in vacuo to give the title compound as a yellow oil (0.5g). T.l.c. (System C 39:10:2) Rf 0.49.

Intermediate 10

N-[4-[3-[[6-[[2-[4-Hydroxy-(2-hydroxyethyl)phenyl]-2-oxoethyl](phenylmethyl)amino]hexyl]oxy]propyl]-phenyl]butanesulphonamide

2-Bromo-1-[4-hydroxy-3-(2-hydroxyethyl)phenyl]ethanone (0.25g), Intermediate 9 (0.45g) and DEA - (0.14g) in DMF (10mℓ) were stirred together at room temperature under nitrogen for 48h. The solution was diluted with water (50mℓ), extracted with ethyl acetate (3 x 50mℓ), dried and evaporated in vacuo to give an oil. Purification by FCC eluting with System C (189:10:1) gave the title compound as a yellow oil (0.51g) T.l.c. (System C 189:10:1) Rf 0.09.

Intermediate 11

(E)-N-[6-[[4-[3-Methoxy-4-(phenylmethoxy)phenyl]-3-butenyl]oxy]hexyl]benzenemethanamine

Intermediate 2 (2.23g) was added dropwise to benzylamine (10ml) stirred at 120° under nitrogen and the solution heated at 120° for a further 2h. The mixture was cooled, poured into 2N hydrochloric acid - (100ml) and extracted with dichloromethane (2 x 75ml). The organic layer was washed with 2N hydrochloric acid, 8% sodium bicarbonate solution (75ml), dried and concentrated in vacuo at 40° to afford the title compound as a pale yellow oil (2.32g). T.l.c. (System C 39:10:1) Rf 0.41.

Intermediate 12

(E)-1-(3,4-Dihydroxyphenyl)-2-[[6-[[4-[3-methoxy-4-(phenylmethoxy)phenyl]-3-butenyl]oxy]hexyl]-(phenylmethyl)amino]ethanone

A mixture of 2-chloro-1-(3,4-dihydroxyphenyl)ethanone (0.56g), Intermediate 11 (1.42g), DEA (1.16g) and potassium iodide (0.1g) was stirred in DMF (10mℓ) under nitrogen for 20h. The solvent was removed in vacuo at 50° and the residual oil partitioned between ethyl acetate (75mℓ) and 2N hydrochloric acid - (75mℓ). The aqueous phase was extracted with ethyl acetate (25mℓ), the combined organic layers washed successively with 8% sodium bicarbonate solution (50mℓ) and pH 7 buffer solution (75mℓ) and then dried. The solution was evaporated onto 'flash' silica, and the resulting impregnated material was purified by FCC eluting with System A (1:4) yielding the title compound as a viscous pale yellow oil (1.17g) T.l.c. (System A 1:4) Rf 0.43.

Intermediate 13

N,N-Diethyl-4-[4-[[6-[(phenylmethyl)amino]hexyl]oxy]-1-butynyl]benzamide

A mixture of N,N-diethyl-4-iodobenzamide (5.79g) , N-[6-[(3-butynyl)oxy]hexyl]benzenemethanamine - (4.95g), BTPC (130mg) and copper (I) iodide (80mg) in diethylamine (100mℓ) was stirred under nitrogen at room temperature for 16h. The solvent was evaporated and the residue was partitioned between ethyl acetate (200mℓ) and 8% aqueous sodium bicarbonate (200mℓ). The organic layer was washed with water - (50mℓ) and brine (50mℓ), dried and concentrated to an oil (9.7g) which was purified by FCC eluting with ethyl acetate-triethylamine (100:1) to give the title compound as a red oil (4.60g). T.l.c. (EtOAc + few drops $Et_3N$) Rf 0.15.

Intermediate 14

N,N-Diethyl-4-[4-[[6-[[2-(1,2-dihydro-8-hydroxy-2-oxoquinolin-5-yl)-2-hydroxyethyl](phenylmethyl)amino]-hexyl]oxy]butyl]benzamide

A solution of 5-oxiranyl-8-(phenylmethoxy)-2(1H)-quinolinone (850mg) and Intermediate 13 (1.26g) in methanol (20mℓ) was stirred at reflux under nitrogen for 18 h. The solvent was evaporated and the residual oil was purified by FCC eluting with System E (4:1) → ethyl acetate to give a yellow oil. The oil in ethanol - (20mℓ) was hydrogenated over pre-reduced 10% PdO-C (100mg). The catalyst was removed by filtration through hyflo, the ethanol was evaporated and the product was purified by FCC eluting with System C - (80:20:1) to give the title compound as a pale yellow solid (130mg) m.p. 101-103°.

Example 1

(a) 5-[1-Hydroxy-2-[[6-[3-[4-(methylthio)phenyl]propoxy]hexyl]amino]ethyl]-1,3-benzenediol, benzoate (salt)

Intermediate 1 (2.1g) was added to a solution of 5-(2-amino-1-hydroxyethyl)benzene-1,3-diol (1.0g) and DEA (1.55g) in DMF (25mℓ) at 70°. The mixture was heated at 75° for 3h, evaporated under reduced pressure and the residue was purified by FCC eluting with System C (80:20:1) to give a colourless gum. The gum - (0.7g) in chloroform (10mℓ) was treated with benzoic acid (0.3g) and chloroform was evaporated. The residue was triturated with ether and dried to give the titlecompound as a beige friable solid (0.7g). T.l.c. - (System C 80:20:1) Rf 0.2.

Analysis Found: C,66.0; H,7.5; N,2.5.

$C_{24}H_{35}NO_4S.C_7H_6O_2.0.5H_2O$ requires C,65.9; H,7.5; N,2.5%.

Similarly were prepared:-

(b) Methyl 4-[3-[[6-[[2-(3,5-dihydroxyphenyl)-2-hydroxyethyl]amino]hexyl]oxy]propyl]benzoate, benzoate (salt) as a beige solid (1.0g) m.p. 72-75°.

Analysis Found: C,67.07.32.4.

$C_{25}H_{35}NO_6.C_7H_6O_2.0.3H_2O$ requires C,67.17.32.4%.
From 5-(2-amino-1-hydroxyethyl)benzene-1,3-diol (0.85g), methyl 4-[3-[(6-bromohexyl)oxy]propyl]benzoate - (1.5g) and DEA (1.3g) after a reaction time of 2h.

(c) 4-[4-[[6-[[2-(3,5-Dihydroxyphenyl)-2-hydroxyethyl]amino]hexyl]oxy]butyl]-N,N-diethylbenzamide, benzoate (salt) as a white friable solid (0.5g) T.l.c. (T-ET-A, 80:20:1) Rf 0.2.
Analysis Found: C,68.58.04.0.
$C_{29}H_{44}N_2O_5.1.25C_7H_6O_2.0.5H_2O$ requires C,68.48.14.4%.
From 5-(2-amino-1-hydroxyethyl)benzene-1, 3-diol (0.54g), 4-[4-[(6-bromohexyl)oxy]butyl]-N,N-diethylbenzamide (0.9g) and DEA (0.78g) after a reaction time of 90min.

Example 2

5-[2-[[6-[4-(4-Aminophenyl)butoxy]hexyl]amino]-1-hydroxy-ethyl]-1,3-benzenediol

A solution of Intermediate 5 (3.2g) in ether (40ml) and THF (10ml) was hydrogenated over 10% Pd-C - (0.2g) and 5% Pt-C (0.2g). The mixture was filtered and evaporated to leave a buff solid. Purification by FCC eluting with System F (90:10:1) gave the title compound as a cream solid (1.1g) m.p. 157-159°. T.l.c. (System G 90:10:1) Rf 0.15.

Example 3

N-[4-[3-[[6-[[2-(3,5-Dihydroxyphenyl)-2-hydroxyethyl]amino]hexyl]oxy]propyl]phenyl]methanesulphonamide

A solution of Example 7 (0.50g) in absolute ethanol (10mℓ) was hydrogenated over 10% Pd-C(0.2g) in absolute ethanol (10mℓ). The mixture was filtered and the filtrate evaporated to yield the title compound as an off-white foam (0.43g) m.p. 66-69°.
Analysis Found: C,59.557.705.51.
$C_{24}H_{36}N_2O_6S.0.25H_2O$ requires C,59.417.585.78%.

Example 4

5-[1-Hydroxy-2-[[6-[4-(4-hydroxy-3-methoxyphenyl)butoxy]hexyl]amino]ethyl]-1,3-benzenediol, benzoate - (salt)

A solution of Intermediate 6 (1.0g) in absolute ethanol (50mℓ) was hydrogenated over 10% PdO-C - (0.25g) in absolute ethanol (15ml). The mixture was filtered, benzoic acid (0.25g) added to the filtrate and the solvent evaporated to yield a viscous oil. Trituration with dry ether followed by evaporation gave the title compound as an off-white foam (0.84g) T.l.c. (System C 39:10:1) Rf 0.20.
Analysis Found: C,66.557.412.36.
$C_{25}H_{37}NO_6.1.3C_7H_6O_2.0.5H_2O$ requires C,66.417.662.42%.

Example 5

5-[1-Hydroxy-2-[[1-methyl-5-[3-[4-(1-pyrrolidinyl)phenyl]propoxy]pentyl]amino]ethyl]-1,3-benzenediol

A mixture of α-(aminomethyl)-3,5-bis(phenylmethoxy)benzenemethanol (0.43g) and Intermediate 7 - (0.38g) was stirred and refluxed in toluene (25mℓ) and a Dean-Stark apparatus for 30min when water ceased to separate. The solution was cooled, diluted with absolute ethanol (20mℓ) and hydrogenated over a pre-reduced 10% PdO-C and 5% PtO-C (0.15g) catalyst mixture in absolute ethanol (10mℓ). The catalyst was removed by filtration through 'hyflo', the solvent was evaporated in vacuo at 40° and the residual product purified by FCC eluting with System C (39:10:1) yielding the title compound as a cream foam -

(0.26g) m.p. 57-60°.
Analysis Found: C,71.20; H,8.84; N,6.07.
$C_{27}H_{40}N_2O_4$ requires C,71.02; H,8.83; N,6.13%.

Example 6

5-[1-Hydroxy-2-[[6-[2-[4-(1-pyrrolidinyl)phenyl]ethoxy]hexyl]amino]ethyl]-1,3-benzenediol, (E) butenedioate - (1:1) (salt)

Intermediate 8 (1.3g) was hydrogenated over pre-reduced 10% PdO-C (50% aqueous paste, 400mg) in ethanol (20mℓ) and THF (5mℓ). The catalyst was removed by filtration through hyflo, the ethanol was evaporated and the residue was partitioned between 8% sodium bicarbonate (25mℓ) and ethyl acetate - (25mℓ). The aqueous layer was re-extracted with ethyl acetate (25mℓ), and the combined organic extracts were washed with sodium bicarbonate (25mℓ) and brine (25mℓ), dried and concentrated to a yellow oil which was purified by FCC eluting wtih System H (98:2:1) to give a pale yellow oil (450mg). A solution of the oil (420mg) and fumaric acid (120mg) in methanol (10mℓ) was concentrated to a foam which was triturated several times with ether to give the title compound as a beige solid (450mg) m.p. 49-52°.
Analysis Found: C,61.39; H,7.32; N,4.43.
$C_{26}H_{38}N_2O_4$.1.25 $C_4H_4O_4$.$H_2O$ requires C,61.47; H,7.49; N,4.62%.

Example 7

(Z)-N-[4-[3-[[6-[[2-(3,5-Dihydroxyphenyl)-2-hydroxyethyl]amino]hexyl]oxy]-1-propenyl]phenyl]-methanesulphonamide

(Z)-N-[4-[3-[(6-Bromohexyl)oxy]-1-propenyl]phenyl]methanesulphonamide (1.56g) was added portionwise over 10 min to a stirred solution of 5-(2-amino-1-hydroxyethyl)-1,3-benzenediol (1.01g) and DEA - (1.55g) in dry DMF (20ml) heated to 80° under nitrogen. When the addition was complete, the mixture was stirred at 80° for 2h, cooled and evaporated onto 'flash' silica. The impregnated material was purified by FCC eluting with System C (39:10:1) to give the title compound as a pale brown powder which was dried in vacuo at 50° for 6h (0.75g) m.p. 79-82°.
Analysis Found: C,61.567.535.67.
$C_{24}H_{34}N_2O_6S$.0.2 $C_7H_8$ requires C,61.367.225.64%.

Example 8

5-[1-Hydroxy-2-[[6-(4-phenylbutoxy)-4-hexynyl]amino]ethyl]-1,3-benzenediol, benzoate (salt)

A solution of 5-(2-amino-1-hydroxyethyl)-1,3-benzenediol (0.5g), [[4-(6-iodo-2-hexynyl)oxy]butyl]benzene (1.0g), DEA (0.7g) and DMF (15ml) was heated at 70-75° for 2h, poured into aqueous sodium bicarbonate - (1M; 100ml) and extracted with ethyl acetate (3×50ml). The dried extract was evaporated and the residue was purified on a column of silica (90ml) eluted with System F (90:10:1) to give an orange oil (0.11g). The oil in chloroform (10ml) was treated with benzoic acid (0.05g) and the chloroform was evaporated. The residue was triturated with ether and dried to give the title compound as an orange foam (0.11g). T.l.c. - (System G 90:10:1) Rf 0.15.
Analysis Found: C,66.5; H,6.9; N,2.6
$C_{24}H_{31}NO_4$.$C_7H_6O_2$.2$H_2O$ requires C,67.0; H,7.4; N,2.5%

Example 9

N-[4-[3-[[6-[[2-Hydroxy-2-[4-hydroxy-3-[(2-hydroxyethyl)]phenyl]ethyl]amino]hexyl]oxy]propyl]phenyl]-butanesulphonamide, benzoate (salt)

A solution of Intermediate 10 (0.50g) in absolute ethanol (25mℓ) was hydrogenated over a mixture of pre-reduced 10% Pd-C (120mg) and 5% Pt-C (120mg) catalysts in absolute ethanol (5mℓ). The mixture was filtered through hyflo and evaported in vacuo to give an oil. Purification by FCC eluting with System C - (64:10:1) gave a colourless oil which was dissolved in chloroform-methanol and treated with benzoic acid - (0.06g). The solution was evaporated in vacuo and triturated with diethyl ether to give the title compound as a cream foam (0.18g). T.l.c. (System C 39:10:1) Rf 0.15.
Analysis Found: C,63.158.154.1.
$C_{29}H_{46}N_2O_6.C_7H_6O_2.0.5H_2O$ requires C,63.47.84.1%.

Example 10

4-[1-Hydroxy-2-[[6-[4-(4-hydroxy-3-methoxyphenyl)butoxy]hexyl]amino]ethyl]-1,2-benzenediol, acetate (salt)

A solution of Intermediate 12 (1.1g) in absolute ethanol (25mℓ) containing glacial acetic acid (0.2mℓ) was hydrogenated over a pre-reduced 10% PdO-C (0.2g, dry) and 5% PtO (0.2g) catalyst mixture. The catalyst was removed by filtration through 'hyflo', the solvent evaporated in vacuo at 40° and the residual dark oil triturated with dry ether to afford the title compound as a dark-grey hygroscopic glass (0.45g), t.l.c. (System C 39:10:1) Rf 0.13.
Analysis Found: C,62.51; H,8.21; N,2.61;
$C_{25}H_{37}NO_6.C_2H_4O_2.0.5H_2O$ requires C,62.77; H,8.20; N,2.71%

Example 11

N,N-Diethyl-4-[4-[[6-[[2-(1,2-dihydro-8-hydroxy-2-oxoquinolin-5-yl)-2-hydroxyethyl]amino]hexyl]oxy]butyl]-benzamide, hydrochloride

Intermediate 14 (120mg) in chloroform (2mℓ) was concentrated in vacuo to a gum which was hydrogenated in ethanol (10mℓ) over pre-reduced 10% Pd-C (25mg). The catalyst was removed by filtration through hyflo and the ethanol was evaporated under vacuum to leave a solid which was triturated with dry ether to give the title compound as an off-white powder (105mg) m.p. 60-80° (hygroscopic). T.l.c. (System C 80:20:1) Rf 0.07.
Analysis Found: C,62.81; H,8.01; N,6.62; Cl,7.27.
$C_{32}H_{45}N_3O_5.1.25HCl.H_2O$ requires C,62.467.906.83;Cl,7.20%.

xample 12

5-[1-Hydroxy-2-[[6-[3-[4-(methylthio)phenyl]propoxy]hexyl]amino]ethyl]-1,3-benzenediol, 3-hydroxy-2-naphthalenecarboxylate (1:1) (salt)

A solution of 5-[1-hydroxy-2-[[6-[3-[4-(methylthio)phenyl]propoxy]hexyl]amino]ethyl]-1,3-benzenediol (422mg) and 3-hydroxy-2-naphthalenecarboxylic acid (190mg) in methanol (5ml) was concentrated in vacuo and the residual oil was triturated with ether and hexane to give the title salt as a dark foam (470mg), δ ($d_4$-MeOH) 1.33-1.75,m and 1.82,m,>10H, $-(CH_2)_6O(CH_2)_3-$; 2.41,s,3H,$-SCH_3$; 2.60,t,2H,-CHz$_2$Ph; 2.94-3.19,m,4H, $-CH_2NHCH_2-$; 3.38,tx2,4H,$-CH_2OCH_2-$; 4.80,m,-CHOH; 6.21,t,1H and 6.37,d,2H, resorcinol aromatic CH; 7.05-7.25,m,6H,7.38,td,1H,7.61,d, 1H, 7.78,d,1H and 8.42,s,1H, Ph and naphthalene aromatic CH.
Analysis Found: C,64.777.302.34.
$C_{23}H_{35}NO_4S.C_{11}H_8O_3.1.05H_2O$ requires C,64.957.23,N,2.23%
Water Analysis 3.01%w/w.

The following are examples of suitable formulations of compounds of the invention. The term "active ingredient" is used herein to represent a compound of the invention.

Tablets (Direct Compression)

| | mg/tablet |
|---|---|
| Active ingredient | 2.0 |
| Microcrystalline Cellulose USP | 196.5 |
| Magnesium Stearate BP | 1.5 |
| Compression weight | 200.0 |

The active ingredient is sieved through a suitable sieve, blended with the excipients and compressed using 7mm diameter punches.

Tablets of other strengths may be prepared by altering the ratio of active ingredient to microcrystalline cellulose or the compression weight and using punches to suit.

The tablets may be film coated with suitable film forming materials, such as hydroxypropylmethylcellulose, using standard techniques. Alternatively the tablets may be sugar coated.

Metered Dose Pressurised Aerosol (Suspension Aerosol)

| | mg/metered dose | Per can |
|---|---|---|
| Active ingredient micronised | 0.100 | 26.40mg |
| Oleic Acid BP | 0.100 | 2.64mg |
| Trichlorofluoromethane BP | 23.64 | 5.67g |
| Dichlorodifluoromethane BP | 61.25 | 14.70g |

The active ingredient is micronised in a fluid energy mill to a fine particle size range. The oleic acid is mixed with the trichlorofluoromethane at a a temperature of 10-15°C and the micronised drug is mixed into the solution with a high shear mixer. The suspension is metered into aluminium aerosol cans and suitable metering valves delivering 85mg of suspension are crimped onto the cans and the dichlorodifluoromethane is pressure filled into the cans through the valves.

Inhalation Cartridges

| | | mg/cartridge |
|---|---|---|
| Active ingredient micronised | | 0.200 |
| Lactose BP | to | 25.0 |

The active ingredient is micronised in a fluid energy mill to a fine particle size range prior to blending with normal tabletting grade lactose in a high energy mixer. The powder blend is filled into No. 3 hard gelatin capsules on a suitable encapsulating machine. The contents of the cartridges are administered using a powder inhaler such as the Glaxo Rotahaler.

**Claims**

1. Compounds of the general formula (I)

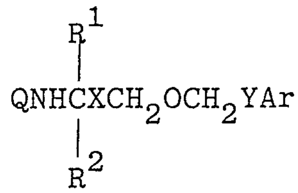

(I)

wherein

Ar represents a phenyl group optionally substituted by one or more substituents selected from halogen atoms, or the groups $C_{1-6}$alkyl, $C_{1-6}$alkoxy, nitro, $-(CH_2)_qR$ (where R is hydroxy, $-NR^3R^4$ (where $R^3$ and $R^4$ each represent a hydrogen atom or a $C_{1-4}$ alkyl group, or $-NR^3R^4$ forms a saturated heterocyclic amino group which has 5-7 ring members and optionally contains in the ring one or more atoms selected from -O-or -S-or a group -NH-or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$ alkoxy or $-NR^3R^4$), $-SR^9$ (where $R^9$ is a hydrogen atom, or a $C_{1-4}$ alkyl or phenyl group), $-SOR^9$, $-SO_2R^9$, or -CN, and q represents an integer from 0 to 3], $-(CH_2)_rR^{10}$ (where $R^{10}$ is a $C_{1-4}$ alkoxy group and r represents an integer from 1 to 3) or $-O(CH_2)_tR^{11}$ (where $R^{11}$ represents a hydroxy or $C_{1-4}$ alkoxy group and t is an integer 2 or 3), or Ar is a phenyl ring substituted by an alkylenedioxy group of formula $-O(CH_2)_p$ O-where p represents an integer 1 or 2;

Q represents a group of formula

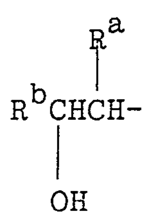 or 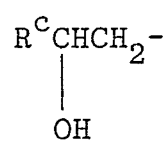

where $R^a$ represents a hydrogen atom or a $C_{1-3}$ alkyl group, $R^b$ represents

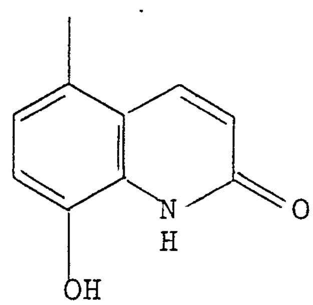 or 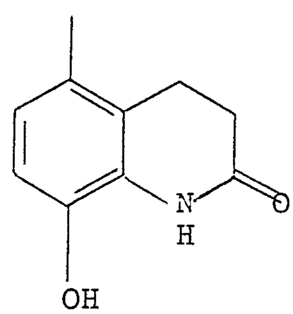

and $R^c$ represents

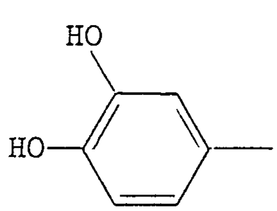 , 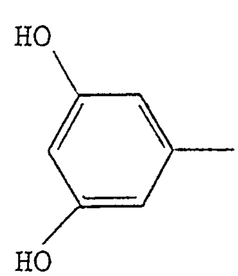 or 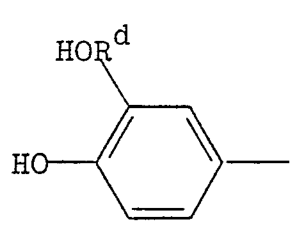

where $R^d$ represents a straight or branched $C_{2-3}$ alkylene chain; $R^1$ and $R^2$ each represent a hydrogen atom or a $C_{1-3}$ alkyl group with the proviso that the sum total of carbon atoms in $R^1$ and $R^2$ is not more than 4;

X represents a $C_{1-7}$ alkylene, $C_{2-7}$ alkenylene or $C_{2-7}$alkynylene chain; and
Y represents a bond or a $C_{1-6}$ alkylene, $C_{2-6}$ alkenylene or $C_{2-6}$ alkynylene chain with the proviso that the sum total of carbon atoms in X and Y is 2-10, and when X represents $C_{1-7}$ alkylene and Y represents a bond or $C_{1-6}$alkylene, then the group Ar does not represent an unsubstituted phenyl group or a phenyl group substituted by one or two substituents selected solely from halogen atoms or $C_{1-3}$ alkyl or $C_{1-3}$ alkoxy groups or an alkylenedioxy group $-O(CH_2)_pO-$; and physiologically acceptable salts and solvates thereof.

2. Compounds as claimed in claim 1 in which the sum total of carbon atoms in the chains X and Y is 5, 6 or 7.

3. Compounds as claimed in claim 1 or 2 in which X is $-(CH_2)_3$-or $-(CH_2)_4$-, and Y is $-CH_2$-, $-(CH_2)_2$-or $-(CH_2)_3$-.

4. Compounds as claimed in any of claims 1 to 3 in which $R^1$ and $R^2$ are both hydrogen atoms, or $R^1$ is a hydrogen atom and $R^2$ is a $C_{1-3}$ alkyl group.

5. Compounds as claimed in any of claims 1 to 4 in which Q represents $R^c\underset{OH}{CH}CH_2$-where $R^c$ represents

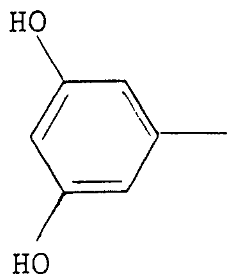

6. Compounds as claimed in any of claims 1 to 4 in which Ar is phenyl; or Ar is phenyl substituted by one or more substituents selected from chlorine, bromine, iodine, fluorine, methyl, ethyl, methoxy, ethoxy, $-(CH_2)_qR$ [where R is hydroxy, amino, methylamino, ethylamino, dimethylamino, diethylamino, morpholino, piperidino, pyrrolidino, piperazino, N-methylpiperazino, $NHCOR^6$ (where $R^6$ is hydrogen, $C_{1-4}$alkyl, $C_{1-4}$ alkoxy, phenyl, amino or N,N-dimethylamino), $-N(CH_3)COCH_3$, $-NR^5SO_2R^7$ (where $R^5$ represents a hydrogen atom or a methyl group, and $R^7$ represents phenyl, methyl, butyl, amino or dimethylamino), -COOH, $-COOCH_3$, $COOCH_2CH_2CH_3$, $-CONH_2$, $-CON(CH_3)_2$, $-CON(CH_2CH_3)_2$, $-CON(CH_2CH_2CH_3)_2$,

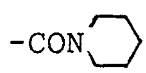 ,

$-SR^9$ (where $R^9$ is methyl, ethyl or phenyl), $-SOCH_3$, $-SO_2CH_3$, or CN and q is zero, 1, 2 or 3], $-NO_2$, $-CH_2OCH_3$, $-(CH_2)_3OCH_3$, $-O(CH_2)_2OH$, $-(OCH_2)_3OH$, $-O(CH_2)_2OCH_3$, or $-O(CH_2)_2OCH_2CH_3$.

7. Compounds as claimed in claim 6 in which Ar is phenyl or phenyl substituted by methoxy and/or hydroxy, or by a group selected from amino, a 5-7 membered heterocyclic amino group, $-NHSO_2R^7$ (where $R^7$ is $C_{1-4}$ alkyl), $-COR^8$ (where $R^8$ is $C_{1-4}$ alkoxy, or $NR^3R^4$ where $R^3$ and $R^4$ are $C_{1-4}$ alkyl), or $-SR^9$ (where $R^9$ is $C_{1-4}$ alkyl).

8. The compounds:
5-[1-hydroxy-2-[[1-methyl-5-[3-[4-(1-pyrrolidinyl) phenyl]propoxypentyl]amino]ethyl]-1,3-benzenediol;
5-[1-hydroxy-2-[[6-[3-[4-(methylthio)phenyl]propoxy]hexyl]aminoethyl]-1,3-benzenediol;
4-[4-[[6-[[2-(3,5-dihydroxyphenyl)-2-hydroxyethyl]amino]hexyl]oxy]-butyl]-N,N-diethylbenzamide;
5-[1-hydroxy-2-[[6-[2-[4-(1-pyrrolidinyl)phenyl]ethoxy]hexyl]amino]ethyl]-1,3-benzenediol;
and physiologically acceptable salts and hydrates thereof.

9. A process for the preparation of compounds as claimed in any of claims 1 to 8 or a physiologically acceptable salt of solvate thereof which comprises:

(1a) for the preparation of a compound of formula (I) in which $R^1$ is a hydrogen atom, alkylating an amine of general formula (II)
$QNR^{12}R^{13}$
(where $R^{12}$ is a hydrogen atom or a protecting group, $R^{13}$ is a hydrogen atom, and Q is

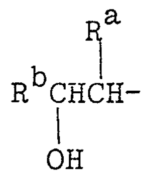 or 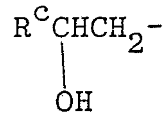

where $R^a$ , $R^b$ and $R^c$ are as defined in claim 1, with any hydroxyl group(s) in $R^b$and $R^c$ optionally protected) with an alkylating agent of formula (III)

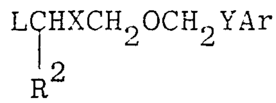 (III)

(wherein L represents a leaving group and $R^2$, X, Y and Ar are as defined in claim 1) followed, if necessary, by removal of any protecting group present; or

(1b) for the preparation of a compound of formula (I) in which $R^1$ is a hydrogen atom, alkylating an amine of general formula (II) as defined above except that $R^{13}$ is a hydrogen atom or a group convertible thereto under the reaction conditions, with a compound of general formula (IV):

$R^2COXCH_2OCH_2YAr$ (IV)

(wherein $R^2$, X, Y and Ar are as defined in claim 1) in the presence of a reducing agent followed, if necessary, by removal of any protecting groups present;

or

(2) deprotection of a protected intermediate of general formula (VI)

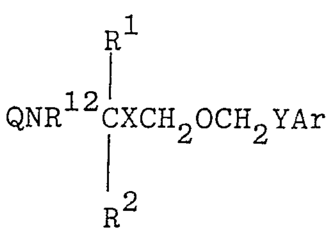 (VI)

(where $R^1$, $R^2$, X, Y and Ar are as defined in claim 1, $R^{12}$ is a hydrogen atom or a protecting group, and

Q is 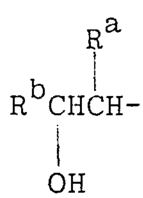 or 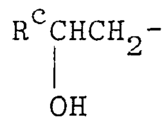

where $R^a$, $R^b$ and $R^c$ are as defined in claim 1, with any hydroxyl group(s) and $R^b$ and $R^c$ optionally protected, and either $R^{12}$ is a protecting group and/or at least one of the hydroxyl group(s) in $R^b$ or $R^c$ is protected); or

(3) reducing an intermediate of general formula (VII)

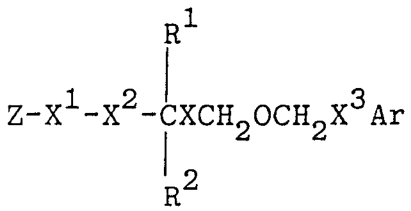

(VII)

(where Z represents $R^b$ and $R^c$ as defined in claim 1 with any hydroxyl group(s) in $R^b$ or $R^c$ optionally protected
$X^1$ is -CH(OH) or a group convertible thereto by reduction;
$X^2$ is $-CHR^aNR^{12}$ (when Z is $R^b$) or $-CH_2NR^{12}$ (when Z is $R^c$) (where $R^{12}$ is as defined above) or a group convertible thereto by reduction, $X^3$ is Y (where Y is a bond or a $C_{1-6}$ alkylene chain) or a group convertible thereto by reduction, and Ar is as defined in claim 1 or a group convertible thereto by reduction;
at least one of $X^1$, $X^2$, $X^3$ representing a reducible group and/or Ar containing a reducible group followed, if necessary, by removal of any protecting group present; and
if desired, converting the resulting compound of general formula (I) or a salt thereof into a physiologically acceptable salt of solvate thereof.

10. A pharmaceutical composition comprising at least one compound of general formula (I) as defined in any of claims 1 to 8 or a physiologically acceptable salt or solvate thereof, together with a physiologically acceptable carrier or excipient.

Claims for the following Contracting States:AT,GR,ES

1. A process for the preparation of compounds of the general formula (I)

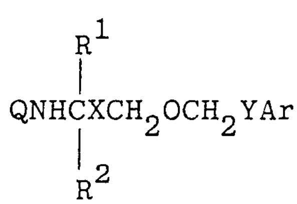

(I)

wherein
Ar represents a phenyl group optionally substituted by one or more substituents selected from halogen atoms, or the groups $C_{1-6}$alkyl, $C_{1-6}$alkoxy, nitro, $-(CH_2)_qR$ (where R is hydroxy, $-NR^3R^4$ (where $R^3$ and $R^4$ each represent a hydrogen atom or a $C_{1-4}$ alkyl group, or $-NR^3R^4$ forms a saturated heterocyclic amino group which has 5-7 ring members and optionally contains in the ring one or more atoms selected from -O- or -S-or a group -NH-or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$ alkoxy or $-NR^3R^4$), $-SR^9$ (where $R^9$ is a hydrogen atom, or a $C_{1-4}$ alkyl or phenyl group), $-SOR^9$, $-SO_2R^9$, or -CN, and q represents an integer from 0 to 3], $-(CH_2)_rR^{10}$ (where $R^{10}$ is a $C_{1-4}$ alkoxy group and r represents an integer from 1 to 3) or $-O(CH_2)_tR^{11}$ (where $R^{11}$ represents a hydroxy or $C_{1-4}$ alkoxy group and t is an integer 2 or 3), or Ar is a phenyl ring substituted by an alkylenedioxy group of formula $-O(CH_2)_p$ O-where p represents an integer 1 or 2;
Q represents a group of formula

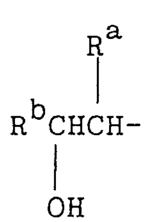

or

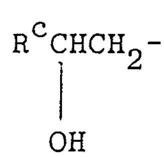

where $R^a$ represents a hydrogen atom or a $C_{1-3}$ alkyl group, $R^b$ represents

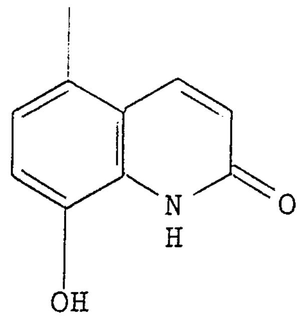

or

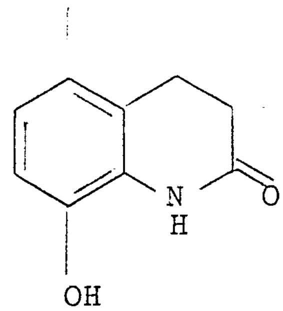

and $R^c$ represents

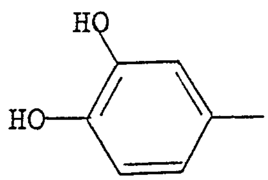

,

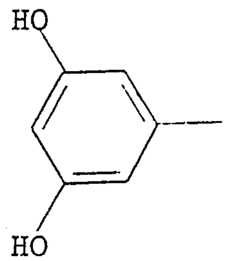

or

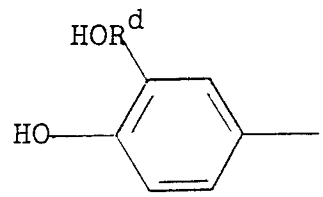

where $R^d$ represents a straight or branched $C_{2-3}$ alkylene chain; $R^1$ and $R^2$ each represent a hydrogen atom or a $C_{1-3}$ alkyl group with the proviso that the sum total of carbon atoms in $R^1$ and $R^2$ is not more than 4;
X represents a $C_{1-7}$ alkylene, $C_{2-7}$ alkenylene or $C_{2-7}$alkynylene chain; and
Y represents a bond or a $C_{1-6}$ alkylene, $C_{2-6}$ alkenylene or $C_{2-6}$ alkynylene chain with the proviso that the sum total of carbon atoms in X and Y is 2-10, and when X represents $C_{1-7}$ alkylene and Y represents a bond or $C_{1-6}$alkylene, then the group Ar does not represent an unsubstituted phenyl group or a phenyl group substituted by one or two substituents selected solely from halogen atoms or $C_{1-3}$ alkyl or $C_{1-3}$ alkoxy groups or an alkylenedioxy group $-O(CH_2)_pO-$; and physiologically acceptable salts and solvates thereof which comprises:

(1a) for the preparation of a compound of formula (I) in which $R^1$ is a hydrogen atom, alkylating an amine of general formula (II)

$QNR^{12}R^{13}$

(where $R^{12}$ is a hydrogen atom or a protecting group, $R^{13}$ is a hydrogen atom, and Q is

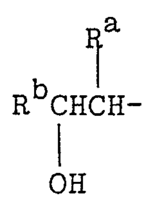

or

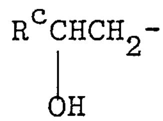

where $R^a$, $R^b$ and $R^c$ are as defined above, with any hydroxyl group(s) in $R^b$and $R^c$ optionally protected) with an alkylating agent of formula (III)

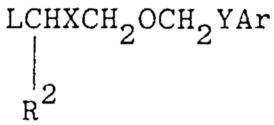

(III)

(wherein L represents a leaving group and $R^2$, X, Y and Ar are as defined in claim 1) followed, if necessary, by removal of any protecting group present; or

(1b) for the preparation of a compound of formula (I) in which $R^1$ is a hydrogen atom, alkylating an amine of general formula (II) as defined above except that $R^{13}$ is a hydrogen atom or a group convertible thereto under the reaction conditions, with a compound of general formula (IV):
$R^2COXCH_2OCH_2YAr$ (IV)
(wherein $R^2$, X, Y and Ar are as defined above) in the presence of a reducing agent followed, if necessary, by removal of any protecting groups present;
or

(2) deprotection of a protected intermediate of general formula (VI)

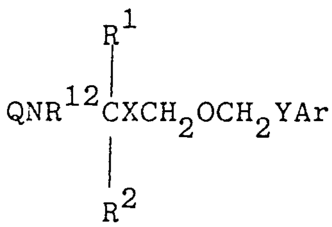

(VI)

(where $R^1$, $R^2$, X, Y and Ar are as defined above, $R^{12}$ is a hydrogen atom or a protecting group, and

Q is 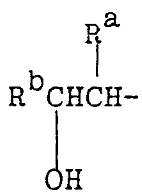 or 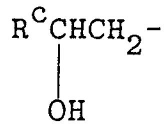

where $R^a$, $R^b$ and $R^c$ are as defined above, with any hydroxyl group(s) and $R^b$ and $R^c$ optionally protected, and either $R^{12}$ is a protecting group and/or at least one of the hydroxyl group(s) in $R^b$ or $R^c$ is protected); or

(3) reducing an intermediate of general formula (VII)

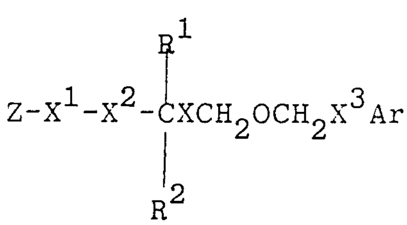

(VII)

(where Z represents $R^b$ and $R^c$ as defined above with any hydroxyl group(s) in $R^b$ or $R^c$ optionally protected
$X^1$ is -CH(OH) or a group convertible thereto by reduction;
$X^2$ is $-CHR^aNR^{12}$ (when Z is $R^b$) or $-CH_2NR^{12}$ (when Z is $R^c$) (where $R^{12}$ is as defined above) or a group convertible thereto by reduction, $X^3$ is Y (where Y is a bond or a $C_{1-6}$ alkylene chain) or a group convertible thereto by reduction, and Ar is as defined above or a group convertible thereto by reduction;
at least one of $X^1$, $X^2$, $X^3$ representing a reducible group and/or Ar containing a reducible group followed, if necessary, by removal of any protecting group present; and
if desired, converting the resulting compound of general formula (I) or a salt thereof into a physiologically acceptable salt or solvate thereof.

2. A process as claimed in claim 1 for the production of compounds in which the sum total of carbon atoms in the chains X and Y is 5, 6, or 7.

3. A process as claimed in claim 1 or 2 for the production of compounds in which X is $-(CH_2)_3$-or $-(CH_2)_4$-, and Y is $-CH_2$-, $-(CH_2)_2$-or $-(CH_2)_3$-.

4. A process as claimed in any of claims 1 to 3 for the production of compounds in which $R^1$ and $R^2$ are both hydrogen atoms, or $R^1$ is a hydrogen atom and $R^2$ is a $C_{1-3}$ alkyl group.

5. A process as claimed in any of claims 1 to 4 for the production of compounds in which Q represents $R^c$ C H $CH_2$-where $R^c$ represents
OH

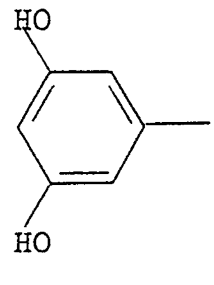

6. A process in any of claim 1 to 4 for the production of compounds in which Ar is phenyl; or Ar is phenyl substituted by one or more substituents selected from chlorine, bromine, iodine, fluorine, methyl, ethyl, methoxy, ethoxy, $-(CH_2)_qR$ [where R is hydroxy, amino, methylamino, ethylamino, dimethylamino, diethylamino, morpholino, piperidino, pyrrolidino, piperazino, N-methylpiperazino, $NHCOR^6$ (where $R^6$ is hydrogen, $C_{1-4}$alkyl, $C_{1-4}$ alkoxy, phenyl, amino or N,N-dimethylamino), $-N(CH_3)COCH_3$, $-NR^5SO_2R^7$ (where $R^5$ represents a hydrogen atom or a methyl group, and $R^7$ represents phenyl, methyl, butyl, amino or dimethylamino), -COOH, $-COOCH_3$, $COOCH_2CH_2CH_3$, $-CONH_2$, $-CON(CH_3)_2$, $-CON(CH_2CH_3)_2$, $-CON(CH_2CH_2CH_3)_2$,

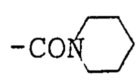 ,

$-SR^9$ (where $R^9$ is methyl, ethyl or phenyl), $-SOCH_3$, $-SO_2CH_3$, or CN and q is zero, 1, 2 or 3], $-NO_2$, $-CH_2OCH_3$, $-(CH_2)_3OCH_3$, $-O(CH_2)_2OH$-, $(OCH_2)_3OH$, $-O(CH_2)_2OCH_3$, or $-O(CH_2)_2OCH_2CH_3$.

7. A process as claimed in claim 6 for the production of compounds in which Ar is phenyl or phenyl substituted by methoxy and/or hydroxy, or by a group selected from amino, a 5-7 membered heterocyclic amino group, $-NHSO_2R^7$ (where $R^7$ is $C_{1-4}$ alkyl), $-COR^8$ (where $R^8$ is $C_{1-4}$ alkoxy, or $NR^3R^4$ where $R^3$ and $R^4$ are $C_{1-4}$ alkyl), or $-SR^9$ (where $R^9$ is $C_{1-4}$ alkyl).

8. A process as claimed in claim 1 for the production of a compound selected from:
5-[1-hydroxy-2-[[1-methyl-5-[3-[4-(1-pyrrolidinyl)phenyl]propoxypentyl]amino]ethyl]-1,3-benzenediol;
5-[1-hydroxy-2-[[6-[3-[4-(methylthio)phenyl]propoxy]hexyl]aminoethyl]-1,3-benzenediol;
4-[4-[[6-[[2-(3,5-dihydroxyphenyl)-2-hydroxyethyl]amino]hexyl]oxy]-butyl]-N,N-diethylbenzamide;
5-[1-hydroxy-2-[[6-[2-[4-(1-pyrrolidinyl)phenyl]ethoxy]hexyl]amino]ethyl]-1,3-benzenediol;
and physiologically acceptable salts and hydrates thereof.